# EUROPÄISCHE PATENTANMELDUNG

(11) **EP 3 637 752 A1**
(43) Veröffentlichungstag der Anmeldung: **15.04.2020**
(21) Anmeldenummer: 19207719.6
(22) Anmeldetag: 12.11.2007
(51) Int. Cl.: H04N 1/00, A61B 90/00

(54) **SYSTEM ZUR WIEDERGABE MEIDZINISCHER BILDER**

(30) Priorität: 11.11.2006 DE 102006053261
(62) Teilanmeldung aus: 07819750.6
(71) Anmelder: VISUS Health IT GmbH, 44801 Bochum (DE)
(72) Erfinder: HOLSTEIN, Jörg, 44869 Bochum (DE); KLEBER, Klaus Dirk, 51377 Leverkusen (DE)
(74) Vertreter: Schneiders & Behrendt PartmbB Rechtsanwälte - Patentanwälte

(57) **Zusammenfassung**

Die Erfindung betrifft ein System zur Wiedergabe medizinischer Bilder in einem Operationssaal, mit einer PACS-Arbeitsstation (1) und mit einer an die PACS-Arbeitsstation angeschlossenen Anzeigeeinrichtung (2) zur Wiedergabe von auf der PACS-Arbeitsstation (1) gespeicherten medizinischen Bildern, wobei die Anzeigeeinrichtung (2) eine großformatige Anzeigefläche aufweist. Um eine effiziente und komfortable Wiedergabe von medizinischen Bildern im Operationssaal zu ermöglichen, schlägt die Erfindung vor, dass die PACS-Arbeitsstation (1) mit einer sterilisierbaren Fernbedienungseinheit (6) verbunden ist, wobei die Fernbedienungseinheit (6) Bedienelemente (9) zur Steuerung der Wiedergabe der medizinischen Bilder über die an die PACS-Arbeitsstation (1) angeschlossene Anzeigeeinrichtung (2) aufweist.

## Beschreibung

Die Erfindung betrifft ein System zur Wiedergabe medizinischer Bilder in einem Operationssaal, mit einer PACS-Arbeitsstation und mit einer an die PACS-Arbeitsstation angeschlossenen Anzeigeeinrichtung zur Wiedergabe von auf der PACS-Arbeitsstation gespeicherten medizinischen Bildern.

Ein Picture Archiving and Communication System (PACS) ist in der Medizin ein Bildarchivierungs- und Kommunikationssystem auf der Basis vernetzter Computer. PACS-Systeme erfassen digitale Bilddaten aller in der Medizin eingesetzter bildgebender Untersuchungsmodalitäten. Die modernen radiologischen Untersuchungsmodalitäten liefern digitale Bilddaten. Nach der Erfassung werden diese digitalen Bilddaten, zusammen mit Informationen über die Identität des untersuchten Patienten, über die klinische Fragestellung und über die Untersuchung selbst auf einem zentralen Serversystem gespeichert. Bei allen heutzutage gebräuchlichen Systemen werden standardisierte Kommunikationsprotokolle und Speicherformate verwendet (z.B. DICOM). Dadurch lassen sich verschiedene PACS-Komponenten und Diagnosegeräte Hersteller unabhängig vernetzen und kombiniert benutzen. An speziellen Arbeitsplatzrechnern, die auch als PACS-Arbeitsstationen oder PACS-Workstations bezeichnet werden, werden die Untersuchungsergebnisse abgerufen. Bilder werden, wenn nötig, digital nachbearbeitet. Nach der Begutachtung mittels einer PACS-Arbeitsstation erstellt der Radiologe einen Befundbericht vor dem Hintergrund der Krankengeschichte, der ebenfalls ins System eingegeben wird. An anderen PACS-Arbeitsstationen, die mit dem Netzwerk verbunden sind, können behandelnde Ärzte die Bilder und den Befundbericht einsehen.

In Operationssälen existieren heutzutage bereits vielfach Videoprojektionsmöglichkeiten, d. h. Anzeigeeinrichtungen mit großformatigen Anzeigeflächen. Diese sind üblicherweise außerhalb der Sterilzone des Operationssaals angeordnet und erzeugen aufgrund der Größe der Anzeigefläche ein vom Operateur aus gut zu erkennendes Bild. Derartige Anzeigeeinrichtungen werden in Operationssälen hauptsächlich genutzt, um mittels Videokameras, Endoskopiesystemen oder Röntgensystemen während einer Operation aufgenommene Bilder wiederzugeben.

Um den Zugriff auf Bilder und Befunde aus der Radiologie während einer Operation zu ermöglichen, ist es des Weiteren bekannt, PACS-Arbeitsstationen der oben beschriebenen Art im Operationssaal einzusetzen. Die Benutzung einer PACS-Arbeitsstation während einer laufenden Operation ist nachteiligerweise nicht sehr praktisch. Die PACS-Arbeitsstation ist außerhalb der Sterilzone des Operationssaals angeordnet, so dass der Operateur zum Einsehen der Bilder ggf. zwischen OP-Tisch und PACS-Arbeitsstation wechseln muss. Außerdem kann der Operateur die (meist nicht sterile) PACS-Arbeitsstation nicht selbst bedienen. Dies muss durch eine Hilfsperson erfolgen. Nachteilig ist aber vor allem, dass der operierende Chirurg die ihn für die Durchführung der Operation interessierenden Bilder nicht unmittelbar während der Operation vom OP-Tisch aus einsehen kann.

Vor diesem Hintergrund ist es die Aufgabe der Erfindung, ein System zu schaffen, dass es dem Operateur während einer laufenden Operation ermöglicht, in komfortabler und effizienter Weise in einem PACS-System gespeicherte radiologische Bilddaten einzusehen.

Diese Aufgabe löst die Erfindung dadurch, dass die an die PACS-Arbeitsstation angeschlossene Anzeigeeinrichtung eine großformatige Anzeigefläche hat, wobei die PACS-Arbeitsstation mit einer Fernbedienungseinheit verbunden ist, welche Bedienelemente zur Steuerung der Wiedergabe der medizinischen Bilder über die an die PACS-Arbeitsstation angeschlossene Anzeigeeinrichtung aufweist.

Gemäß der Erfindung werden die in der PACS-Arbeitsstation gespeicherten Bilder über eine mit der PACS-Arbeitsstation verbundene Anzeigeeinrichtung mit großformatiger Anzeigefläche wiedergegeben. Diese Bilder kann der Operateur vom OP-Tisch aus gut sehen, selbst wenn sich die Anzeigeeinrichtung außerhalb der Sterilzone des Operationssaals befindet. Zur Steuerung der Wiedergabe der medizinischen Bilder dient eine Fernbedienungseinheit, die ihrerseits mit der PACS-Arbeitsstation verbunden ist. Die Fernbedienungseinheit ist sinnvollerweise sterilisierbar ausgebildet, so dass die Fernbedienungseinheit direkt am OP-Tisch angeordnet sein kann. Der Operateur kann die Steuerung der Wiedergabe der medizinischen Bilder sowie die Auswahl der auf der PACS-Arbeitsstation gespeicherten Bilder während der laufenden Operation mittels der Fernbedienungseinheit selbst vornehmen. Eine Hilfsperson zur Bedienung der PACS-Arbeitsstation ist nicht erforderlich. Gemäß der Erfindung ist also die Einsichtnahme in radiologische Bilder, die in einem PACS-System gespeichert sind, im Operationssaal besonders effizient und komfortabel möglich.

Gemäß einer sinnvollen Ausgestaltung des erfindungsgemäßen Systems weist die Fernbedienungseinheit eine Anzeigeeinrichtung mit einer kleinformatigen Anzeigefläche zur Wiedergabe der auf der PACS-Arbeitsstation gespeicherten medizinischen Bilder auf. Die kleinformatige Anzeigefläche kann der Operateur komfortabel nutzen, um die ihn interessierenden Bilder auszuwählen oder um die Anzeige von Bildausschnitten zu steuern. Die zusätzliche kleinformatige Anzeigefläche macht die Benutzung der Fernbedienungseinheit besonders komfortabel, da der Operateur beim "Navigieren" in den auf der PACS-Arbeitsstation gespeicherten Bilddaten nur auf die Fernbedienungseinheit blicken muss, die sich unmittelbar am OP-Tisch befindet. Der Operateur muss also nicht zwischen der mit der PACS-Arbeitsstation verbundenen Anzeigeeinrichtung und der Fernbedienungseinheit hin- und herblicken. Zur Steuerung der Bildwiedergabe kann er sich ganz auf die Fernbedienungseinheit konzentrieren. Für die Fernbedienungseinheit reicht vorteilhafterweise eine Anzeigeeinrichtung mit vergleichsweise geringer Qualität der Bildwiedergabe aus. Die in die Fernbedienungseinheit integrierte Anzeigeeinrichtung soll nur die Steuerung der Bildwiedergabe erleichtern. Die eigentliche Wiedergabe der Bilder mit optimaler Bildqualität erfolgt über die großformatige Anzeigefläche der mit der PACS-Arbeitsstation verbundenen Anzeigeeinrichtung.

Zweckmäßigerweise ist die kleinformatige Anzeigefläche der in die Fernbedienungseinheit integrierten Anzeigeeinrichtung als Tastschirm ausgebildet. Ein Tastschirm (auch als Touchscreen oder Sensorbildschirm bezeichnet) ist bekanntlich ein Bildschirm mit Bereichen, durch deren Berührung Steuersignale erzeugt werden. Die Steuerung kann mit dem Finger oder mit einem geeigneten Zeigestift erfolgen. Bei dem erfindungsgemäßen System hat ein Tastschirm den Vorteil, dass der Operateur durch Antippen der Anzeigefläche die ihn interessierenden Bilder oder Bildbereiche einfach und komfortabel auswählen kann. Der Tastschirm ermöglicht eine besonders intuitive Bedienung. Der Tastschirm kann außerdem dazu genutzt werden, variable Bedienfelder anzuzeigen, die je nach Programmkontext mit unterschiedlichen Funktionen belegt sein können. Sinnvollerweise ist also die Fernbedienungseinheit eingerichtet zur interaktiven Auswahl von medizinischen Bildern oder zur Auswahl von Ausschnitten aus medizinischen Bildern durch Berührung des Tastschirms, wobei die ausgewählten Bilder oder Bildausschnitte dann über die an die PACS-Arbeitsstation angeschlossene Anzeigeeinrichtung wiedergegeben werden.

Um eine möglichst unkomplizierte Bedienung zu gewährleisten, kann die Fernbedienungseinheit des erfindungsgemäßen Systems einige wenige Bedientasten mit fester Funktion aufweisen. So kann die Fernbedienungseinheit z. B. Bedientasten zum Wechsel zwischen medizinischen Bildern, Bildserien und Studien, zur Auswahl von Wiedergabeprotokollen, die Parameter für die Wiedergabe der medizinischen Bilder (so genannte Hängungen) definieren, zur Aktivierung einer vergrößerten oder verkleinerten Wiedergabe der medizinischen Bilder, zur Beeinflussung der Helligkeit und/oder des Kontrastes der Bildwiedergabe und/oder zum Starten und Anhalten der Wiedergabe einer aus mehreren Bildern bestehenden dynamischen Bildserie aufweisen. Damit sind die wesentlichen Funktionen, die bei der Bildwiedergabe im Operationssaal häufig benötigt werden, abgedeckt.

Gemäß einer sinnvollen Weiterbildung des erfindungsgemäßen Systems ist es eingerichtet zur automatischen Anpassung der Seitenverhältnisse bei der Wiedergabe der medizinischen Bilder auf der an die PACS-Arbeitsstation angeschlossenen Anzeigeeinrichtung und/oder auf der Anzeigeeinrichtung der Fernbedienungseinheit, nämlich entsprechend dem Bildseitenverhältnis des jeweils wiedergegebenen medizinischen Bildes, dem Anzeigeseitenverhältnis und dem Punktseitenverhältnis der jeweiligen Anzeigeeinrichtung. Wichtig ist, dass sowohl auf der an die PACS-Arbeitsstation angeschlossenen Anzeigeeinrichtung als auch auf der Anzeigeeinrichtung der Fernbedienungseinheit die Wiedergabe der Bilder so erfolgt, dass die in den Bildern enthaltenen anatomischen Strukturen realitätsgetreu reproduziert werden. Verzerrungen aufgrund unterschiedlicher Seitenverhältnisse bei der Wiedergabe müssen vermieden werden. Zu beachten sind hierbei zum einen das Bildseitenverhältnis, d. h. das Verhältnis von Breite zu Höhe des jeweils wiedergegebenen medizinischen Bildes, das Anzeigeseitenverhältnis, d. h. das Verhältnis von Breite zu Höhe der jeweiligen Anzeigefläche, und das Punktseitenverhältnis der jeweiligen Anzeigeeinrichtung, d. h. das Verhältnis von Breite zu Höhe der einzelnen Bildpunkte. Sämtliche dieser Größen müssen bekannt sein, damit durch entsprechende Korrektur bei der Bildwiedergabe dafür gesorgt werden kann, dass keine Verzerrungen auftreten.

Bei der an die PACS-Arbeitsstation gemäß der Erfindung angeschlossenen Anzeigeeinheit kann es sich um einen großformatigen Bildschirm an sich bekannter Art, insbesondere um einen LCD-, Plasma- oder CRT-Bildschirm handeln. Ebenso eignet sich ein Projektor zur Projektion der medizinischen Bilder auf eine vom Operateur aus gut sichtbare Projektionsfläche. Als Projektor kann ein handelsüblicher Videoprojektor mit ausreichender Auflösung verwendet werden. Ein Projektor hat den Vorteil, dass die Projektionsfläche besonders großformatig ist und damit eine für den Operateur besonders gut erkennbare Bildwiedergabe ermöglicht.

Gemäß einer weiteren sinnvollen Ausgestaltung weist die Fernbedienungseinheit des erfindungsgemäßen Systems Mittel zur Spracherkennung auf. Dies ermöglicht eine Sprachsteuerung der Bildwiedergabe. Der Operateur kann die ihn interessierenden Bilder oder Bildausschnitte durch Sprache interaktiv auswählen. Dies hat den Vorteil, dass er beide Hände für die durchzuführende Operation frei hat.

Ein Ausführungsbeispiel der Erfindung wird im Folgenden anhand der Zeichnungen näher erläutert. Es zeigen:
- Fig. 1: Blockdiagrammdarstellung des erfindungsgemäßen Systems;
- Fig. 2: Ansicht der Fernbedienungseinheit des erfindungsgemäßen Systems.

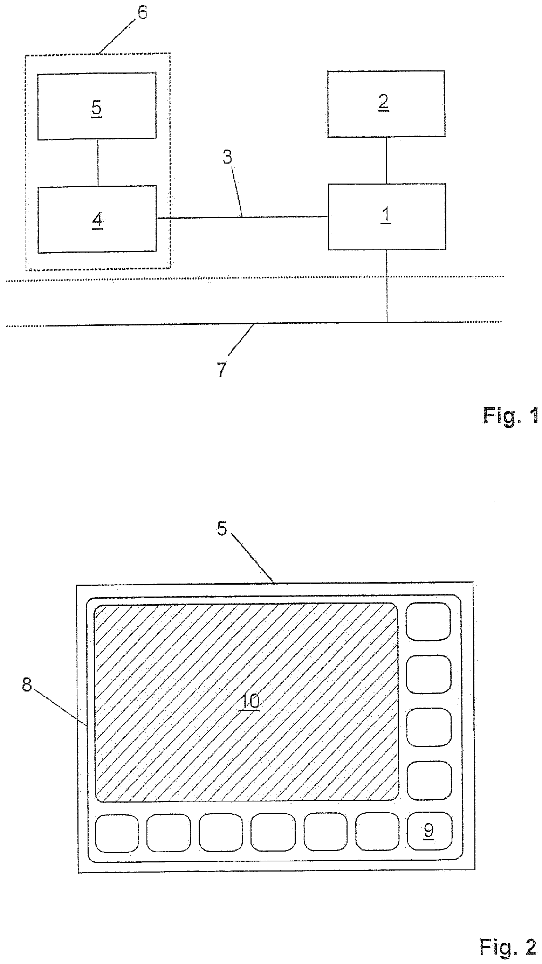

Das in der Fig. 1 dargestellte System dient zur Wiedergabe medizinischer Bilder in einem Operationssaal. Das System umfasst eine PACS-Arbeitsstation 1, auf welcher die wiederzugebenden Bilddaten gespeichert sind. An die PACS-Arbeitsstation 1 ist eine Anzeigeeinrichtung 2 angeschlossen. Bei der Anzeigevorrichtung 2 kann es sich um einen Videoprojektor handeln, der in der Lage ist, medizinische Bilder in hoher Qualität und ausreichender Auflösung zu projizieren. Der Videoprojektor 2 projiziert die medizinischen Bilder auf eine (in der Fig. 1 nicht näher dargestellte) großformatige Projektionsfläche. Dies ermöglicht eine vom OP-Tisch aus gut sichtbare Bildwiedergabe, selbst wenn sich der Videoprojektor 2 und die zugehörige Videoprojektionsfläche außerhalb der Sterilzone des Operationssaals befinden. Eine Projektionsfläche mit einer Diagonalen von 50 cm oder mehr ist auf jeden Fall als großformatig in diesem Sinne anzusehen. Noch größere Projektionsflächen, die sich mit herkömmlichen, kommerziell erhältlichen Videoprojektoren ohne weiteres erzielen lassen, sind von Vorteil. Die PACS-Arbeitsstation 1 ist über eine übliche Netzwerkverbindung 3 mit einem Computer 4 verbunden. Bei dem Computer 4 handelt es sich um einen handelsüblichen OP-PC, d. h. um einen Personalcomputer, der den Anforderungen im Operationssaal entspricht und sterilisierbar ist. An den OP-PC 4 ist ein ebenfalls sterilisierbarer Bildschirm 5 angeschlossen, dessen kleinformatige Anzeigefläche als Tastschirm ausgebildet ist. Der OP-PC 4 bildet zusammen mit dem Monitor 5 eine Fernbedienungseinheit 6, mittels welcher die Wiedergabe der medizinischen Bilder über den an die PACS-Arbeitsstation 1 angeschlossenen Videoprojektor 2 steuerbar ist. Zumindest der Monitor 5 ist unmittelbar am OP-Tisch angeordnet, so dass der Operateur die Bildwiedergabe vom OP-Tisch aus komfortabel und effizient steuern kann. Ein Vorteil der in der Fig. 1 dargestellten Anordnung ist, dass sich diese mit kommerziell erhältlichen Komponenten problemlos realisieren lässt. Für die Fernsteuerungsfunktionalität muss lediglich auf dem OP-PC 4 lauffähige Software bereitgestellt werden. Diese kommuniziert über die Datenverbindung 3 mit einem entsprechenden Softwaremodul, das auf der PACS-Arbeitsstation 1 hierfür installiert ist.

Die PACS-Arbeitsstation 1 ist außerdem mit einem Datennetzwerk 7 verbunden, so dass Zugriff auf die weiteren DV-Systeme im Krankenhaus besteht, beispielsweise auf ein herkömmliches Krankenhausinformationssystem (KIS) sowie auf PACS-Server, auf welchen die medizinischen Bilddaten und Befunde zentral vorgehalten werden. Das in der Fig. 1 dargestellte System kann mit Vorteil in Kombination mit einer herkömmlichen OP-Planungssoftware benutzt werden. So kann beispielsweise die Fernbedienungseinheit 6 dazu genutzt werden, zur Vorbereitung einer Operation automatisch einen Abgleich der Patientendaten (Name, Geburtsdatum etc.) mit dem KIS-System durchzuführen, um den Patientenkontext herzustellen. Dabei werden automatisch (oder teilautomatisch) alle relevanten Bilddaten auf die PACS-Arbeitsstation von einem an das Datennetz 7 angeschlossenen PACS-Server vorgeladen.

Die Fig. 2 zeigt eine schematische Ansicht auf die als Tastschirm ausgebildete Anzeigefläche 8 des Monitors 5. Auf der Anzeigefläche 8 werden mehrere Bedienfelder 9 angezeigt, denen bestimmte Funktionen durch entsprechende Konfiguration zugeordnet sind. Die Bedienfelder 9 können durch Berühren des Tastschirms 8 mit dem Finger aktiviert werden. Somit bilden die Bedienfelder 9 Bedientasten zum Wechsel zwischen medizinischen Bilder, Bildserien und Studien, zur Auswahl von Wiedergabeprotokollen (Hängungen), zur Beeinflussung der Helligkeit und/oder des Kontrastes der Bildwiedergabe sowie zum Starten und Anhalten der Wiedergabe einer aus mehreren Bildern bestehenden dynamischen Bildserie. Des Weiteren ist eine kleinformatige Anzeigefläche 10 vorgesehen, die zur Darstellung einer Übersicht über die gleichzeitig mittels des Videoprojektors 2 großformatig wiedergegebenen medizinischen Bilder darstellt. Der Operateur kann durch Berühren der Anzeigefläche 10 bestimmte ihn interessierende Bilder oder Bildausschnitte wählen sowie den jeweils gewählten Bildausschnitt verschieben. Dementsprechend wird die Wiedergabe der medizinischen Bilder über die PACS-Arbeitsstation 1 gesteuert. Die Anzeigefläche 10 ist kleinformatig. Die Bilddiagonale beträgt vorzugsweise 50 cm oder weniger.

Wichtig ist, dass bei der großformatigen Wiedergabe der medizinischen Bilder über den Projektor 2 und bei der gleichzeitigen Wiedergabe auf der kleinformatigen Anzeigefläche 10 eine Anpassung der Seitenverhältnisse erfolgt, um Verzerrungen zu vermeiden. Die großformatige und die kleinformatige Wiedergabe sollen jeweils die gezeigten anatomischen Strukturen möglichst realitätsnah wiedergeben. Zu beachten ist, dass die großformatige Anzeigefläche, d.h. z. B. die mittels des Videoprojektors 2 angestrahlte Projektionsfläche, und die kleinformatige Anzeigefläche 10 unterschiedliche Seitenverhältnisse haben. Die Projektionsfläche kann beispielsweise ein Seitenverhältnis von 16:9 oder 16:10 haben, während die kleinformatige Anzeigefläche 10 ein Seitenverhältnis von 4:3 hat. Bei der Bildwiedergabe muss eine automatische Anpassung (z.B. durch entsprechende Bildtransformation) erfolgen, um Verzerrungen aufgrund der unterschiedlichen Seitenverhältnisse zu vermeiden. Bei dieser Anpassung sind außerdem die Bildseitenverhältnisse der jeweils wiedergegebenen medizinischen Bilder sowie die Punktseitenverhältnisse der jeweiligen Anzeigeeinrichtungen zu berücksichtigen. Die unterschiedlichen Anzeigeeinrichtungen können unterschiedliche Punktseitenverhältnisse haben. So könnte z. B. die kleinformatige Anzeigefläche des Monitors 5 quadratische Bildpunkte (Pixel) haben, während die Bildpunkte des Videoprojektors 2 rechteckig sind. Dieses Punktseitenverhältnis (auch Pixelmetrik genannt) muss bei der Bildwiedergabe berücksichtigt werden. Ggf. müssen Korrekturen bei der Bildwiedergabe vorgenommen werden, damit die kleinformatige und die großformatige Bildwiedergabe gleichermaßen verzerrungsfrei sind.

## Patentansprüche

1. System zur Wiedergabe medizinischer Bilder in einem Operationssaal, mit einer PACS-Arbeitsstation (1) und mit einer an die PACS-Arbeitsstation (1) angeschlossenen Anzeigeeinrichtung (2) zur Wiedergabe von auf der PACS-Arbeitsstation (1) gespeicherten medizinischen Bildern, **dadurch gekennzeichnet, dass** die Anzeigeeinrichtung (2) eine großformatige Anzeigefläche hat, wobei die PACS-Arbeitsstation (1) mit einer Fernbedienungseinheit (6) verbunden ist, welche Bedienelemente (9) zur Steuerung der Wiedergabe der medizinischen Bilder über die an die PACS-Arbeitsstation (1) angeschlossene Anzeigeeinrichtung (2) aufweist.

2. System nach Anspruch 1, **dadurch gekennzeichnet, dass** die Fernbedienungseinheit (6) eine Anzeigeeinrichtung (5) mit einer kleinformatigen Anzeigefläche (10) zur Wiedergabe der auf der PACS-Arbeitsstation (1) gespeicherten medizinischen Bilder aufweist.

3. System nach Anspruch 2, **dadurch gekennzeichnet, dass** die kleinformatige Anzeigefläche (10) als Tastschirm ausgebildet ist.

4. System nach Anspruch 3, **dadurch gekennzeichnet, dass** die Fernbedienungseinheit (6) eingerichtet ist zur interaktiven Auswahl von medizinischen Bildern oder zur Auswahl von Ausschnitten aus medizinischen Bildern durch Berührung des Tastschirms, wobei die ausgewählten Bilder oder Bildausschnitte über die an die PACS-Arbeitsstation (1) angeschlossene Anzeigeeinrichtung (2) wiedergegeben werden.

5. System nach einem der Ansprüche 1 bis 4, **dadurch gekennzeichnet, dass** die Fernbedienungseinheit Bedientasten (9) zum Wechsel zwischen medizinischen Bildern, Bildserien und Studien, zur Auswahl von Wiedergabeprotokollen, die Parameter für die Wiedergabe der medizinischen Bilder definieren, zur Aktivierung einer vergrößerten oder verkleinerten Wiedergabe der medizinischen Bilder, zur Beeinflussung der Helligkeit und/oder des Kontrastes der Bildwiedergabe und/oder zum Starten und Anhalten der Wiedergabe einer aus mehreren Bildern bestehenden dynamischen Bildserie aufweist.

6. System nach einem der Ansprüche 1 bis 5, **dadurch gekennzeichnet, dass** es eingerichtet ist zur automatischen Anpassung der Seitenverhältnisse bei der Wiedergabe der medizinischen Bilder auf der an die PACS-Arbeitsstation (1) angeschlossenen Anzeigeeinrichtung (2) und/oder auf der Anzeigeeinrichtung (5) der Fernbedienungseinheit (6), nämlich entsprechend dem Bildseitenverhältnis des jeweils wiedergegebenen medizinischen Bildes, dem Anzeigeseitenverhältnis und dem Punktseitenverhältnis der jeweiligen Anzeigeeinrichtung (2, 5).

7. System nach einem der Ansprüche 1 bis 6, **dadurch gekennzeichnet, dass** die an die PACS-Arbeitsstation (1) angeschlossene Anzeigeeinrichtung (2) ein großformatiger Bildschirm, insbesondere ein LCD-, Plasma- oder CRT-Bildschirm, oder ein Projektor zur Projektion der medizinischen Bilder auf eine Projektionsfläche ist.

8. System nach einem der Ansprüche 1 bis 7, **dadurch gekennzeichnet, dass** die Fernbedienungseinheit (6) Mittel zur Spracherkennung aufweist.

9. System nach einem der Ansprüche 1 bis 8, **dadurch gekennzeichnet, dass** die PACS-Arbeitsstation (1) und die daran angeschlossene Anzeigeeinrichtung (2) außerhalb der Sterilzone und die Fernbedienungseinheit (6) innerhalb der Sterilzone des Operationssaals angeordnet sind.
